# EUROPÄISCHE PATENTSCHRIFT

(11) **EP 0 715 860 B1**
(45) Veröffentlichungstag und Bekanntmachung des Hinweises auf die Patenterteilung: **29.09.1999**
(21) Anmeldenummer: 95119204.6
(22) Anmeldetag: 06.12.1995
(51) Int. Cl.: A61M 1/28, A61M 39/18, A61M 39/22

(54) **Vorrichtung zum Steuern eines Fluidverlaufes**
Flow sequence control device
Dispositif pour contrôler la séquence de débit

(30) Priorität: 09.12.1994 DE 4443714
(43) Veröffentlichungstag der Anmeldung: 12.06.1996
(73) Patentinhaber: Fresenius Medical Care Deutschland GmbH, 61350 Bad Homburg v.d.H. (DE)
(72) Erfinder: Heilmann,Klaus, DE-66606 St.Wendel (DE); Herrmann,Josef, DE-66571 Eppelborn (DE); Jessen,Claus Dr, DE-66620 Otzenhausen (DE); Schmidt,Helmut, DE-66649 Oberthal (DE); Schwegmann,Christian Dipl.-Ing, DE-66606 St.Wendel (DE); Zimmermann,Michael, DE-66606 St.Wendel (DE)
(74) Vertreter: Vièl, Georg, Dipl.-Ing.

(56) Entgegenhaltungen:
- EP-A- 0 639 389
- WO-A-88/06895

## Beschreibung

Die Erfindung betrifft eine Vorrichtung zum Steuern eines Fluidverlaufes, bestehend aus einem Gehäuse mit mindestens einem Stutzen zur festen Ankupplung eines Schlauchabschnitts und mindestens einem weiteren Stutzen zur Aufnahme eines Kupplungsteiles oder Konnektionsstücks, wobei die Verbindung zwischen den weiteren Stutzen und dem Kupplungsteil oder dem Konnektionsstück lösbar ist und wobei eine drehbare Anordnung zum Steuern des Fluidverlaufes durch das Gehäuse sowie Mittel zum Abdichten der Stutzen vorgesehen sind.

Vorrichtungen zum Verbinden von mehreren Schlauchabschitten werden in vielen Bereichen der Technik verwendet. Sollen beispielsweise drei Schlauchabschnitte verbunden werden, sind die Vorrichtungen zum Verbinden der Schlauchabschnitte in der Regel als sogenannte Y-Stücke ausgebildet. Durch das Drehen der Y-Stücke werden jeweils zwei Schlauchabschnitte miteinander verbunden.

Derartige Vorrichtungen zum Verbinden von Schlauchabschnitten werden beispielsweise zum Anschluß an den Bauchkatheter eines Patienten bei der Peritonealdialyse verwendet, weshalb die Erfindung im folgenden am Beispiel dieser Anwendung beschrieben werden soll.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyseflüssigkeit ausgetauscht. Zu diesem Zweck ist an dem bauchseitigen Katheterabschnitt des Patienten ein Kupplungsstück angeordnet, über das ein Y-Stück angeschlossen werden kann, das einerseits zu einem Vorratsbeutel für frische Dialyseflüssigkeit und andererseits zu einem Vorratsbeutel für verbrauchte Dialyseflüssigkeit führt.

Ein großes Problem bei der beschriebenen kontinuierlich ambulanten Peritonealdialyse, die auch als CAPD (continuous ambulatory peritoneal dialysis) bezeichnet wird, ist die Vermeidung der Einführung von Keimen in die Bauchhöhle über den durch die Bauchdecke führenden Katheterabschnitt, da diese unweigerlich zu einer Bauchfellentzündung (Peritonitis) führen würden.

Es sind daher in der Vergangenheit verschiedene Verbindungselemente für diese Anwendung vorgeschlagen worden, die einerseits eine schnelle Ankupplung des Y-Stücks an den bauchseitigen Katheterabschnitt ermöglichen, andererseits eine Kontamination der flüssigkeitsführenden Teile des Verbindungssystems und des bauchseitigen Katheterabschnitts erschweren.

Aus der EP-OS 0 098 103 ist eine Katheterkupplung für die ambulante Peritonealdialyse bekannt, bei der ein weibliches Kupplungsstück aus Edelstahl oder Titan am bauchseitigen Katheterabschnitt des Patienten verbleibt, während ein aus einem für medizinische Zwecke zugelassenen Thermoplast gefertigtes männliches Kupplungsstück als Wegwerfteil ausgebildet ist und bei jedem Wechsel der Dialyseflüssigkeit ausgetauscht wird. Die Paßflächen der beiden Kupplungsstücke sind als Luer-Konen ausgebildet. An dem bekannten Kupplungssystem ist nachteilig, daß das weibliche, am bauchseitigen Katheterabschnitt des Patienten verbleibende Kupplungsstück nach den, Austausch der Dialyseflüssigkeit und dem Abnehmen des Y-Stückes zusammen mit dem männlichen Kupplungsteil durch ein gesondert bereitgehaltenes Verschlußmittel verschlossen werden muß, um den bauchseitigen Katheterabschnitt des Patienten zu verschließen und eine Kontamination zu verhindern. Dabei stellt die zum Einführen des üblicherweise stopfenartig ausgebildeten Verschlußmittels notwendige Manipulation des weiblichen Kupplungsstückes eine zusätzliche Kontaminationsquelle dar, die vermieden werden sollte.

Aus der EP-OS 0 073 432 ist eine ähnliche Verbindungsvorrichtung bekannt, bei der ein kanülenartig ausgebildeter Hohldorn eine in einem Kupplungsteil angeordnete Membran durchsticht, um die Verbindung herzustellen. Zusätzlich ist die Verbindungsstelle mit einem ringförmigen, in eine Desinfektionslösung getauchten Schwamm umgeben, der eine Kontamination verhindern soll. An dieser bekannten Verbindungsanordnung ist nachteilig, daß der konstruktive Aufwand relativ hoch ist und die mit einer Desinfektionslösung getränkten Schwämme schwierig zu handhaben, aufwendig herzustellen und nicht beliebig lange lagerbar sind. Desweiteren ist nicht auszuschließen, daß unnötige Mengen an Desinfektionsmittel in die Bauchhöhle des Patienten gelangen.

Aus der US-A-5,057,074 ist ein Verfahren für eine sterile Konnektion bekannt, bei der der Verbindungskatheter zum Vortatsbeutel für die Dialyseflüssigkeit durchschnitten wird und das verbleibende Katheterendstück zusammen mit der kompletten Konnektoranordnung, d.h. Schlauchkupplung am bauchseitigen Katheterteil des Patienten verbleibt. Beim erfolgenden Austausch der Dialyseflüssigkeit wird der verbliebene Katheterabschnitt entfernt und eine neue Ankopplung bzw. Konnektion durchgeführt. An dieser Vorgehensweise ist nachteilig, daß der Patient zwischen zwei Wechseln der Dialyseflüssigkeit den verbliebenen Katheterabschnitt am Körper mit sich führt, was lästig ist. Darüber hinaus kann sich der am Körper des Patienten verbliebene längere Katheterabschnitt beispielsweise in der Kleidung verfangen und dadurch gelöst werden, was ein hohes Kontaminationsrisiko mit sich bringt ebenso wie ein gefährliches und unhygienisches Auslaufen der Dialyseflüssigkeit aus dem Bauchraum.

Aus der US-A-4,821,996 ist ein Konnektor bekannt, der aus einem Gehäuse mit zwei Stutzen zur festen Ankopplung von Schlauchabschnitten und einem dritten Stutzen zum Ankoppeln an einen Bauchkatheter besteht. Der Konnektor ist vorzugsweise zylindrisch ausgebildet und weist eine nur in eine Richtung drehbare Anordnung sowie damit verbundene Mittel zum Blockieren jeweils mindestens eines der das Gehäuse durchlaufenden Flüssigkeitswege auf. Durch Drehen der drehbaren Anordnung um jeweils eine Position werden die bei der Peritonealdialyse erforderlichen Schritte nacheinander ausgeführt. Durch diese Vorrichtung wird die Fehlerhäufigkeit bei der Durchführung der Peritonealdialyse deutlich vermindert, allerdings muß auch bei dieser Vorrichtung der bauchseitige Katheterabschnitt nach dem Austausch der Dialyseflüssigkeit manuell verschlossen werden, was ein erhöhtes Kontaminationsrisiko mit sich bringt.

Der Erfindung liegt somit die Aufgabe zugrunde, eine Vorrichtung zum Steuern eines Fluidverlaufes gemäß dem Oberbegriff zu schaffen, bei der eine Kontamination beim Verschließen einer der Ausgänge weitgehend vermieden werden kann.

Diese Aufgabe wird erfindungsgemäß dadurch gelöst, daß die Mittel zum Abdichten der Stutzen mindestens ein in dem Gehäuse angeordnetes bewegliches Verschlußstück beinhalten und daß die drehbare Anordnung Mittel zum Übertragen einer radialen Bewegung auf das Verschlußstück aufweist.

Die erfindungsgemäße Vorrichtung, die auch als Konnektor bezeichnet wird, kann entweder über ein Kupplungsteil oder auch direkt mit einem Konnektionsstück am Bauch des Patienten verbunden werden. Ausgehend vom Stand der Technik wurde erfindungsgemäß eine drehbare Konnektoranordnung dergestalt weitergebildet, daß eine Kontamination weitgehend vermieden wird. Bei der Peritonealdialyse ist es notwendig, die einzelnen Verfahrensschritte nacheinander auszuführen. So muß zuerst die verbrauchte, aber sterile Dialyselösung abgelassen werden, um etwaige Keime in der Konnektoranordnung wegzuspülen. Dann wird Lösung aus dem Vorratsbeutel über den Konnektor zum Auslaß gespült, um das komplette System mit der frischen, sterilen Lösung vorzuspülen, wobei der dritte Zugang zum Bauchraum verschlossen ist. Erst im dritten Verfahrensschritt wird die frische Dialyselösung in den Bauchraum gefüllt, wobei nun der Abfluß verschlossen ist und durch Weiterdrehen der Katheterkupplung wird der Bauchzugang mit einem integrierten Verschlußstück verschlossen, ohne das Verschlußstück berühren zu müssen.

Erst nach dem Verschließen des Kupplungsteils wird der Konnektor abgeschraubt und zusammen mit dem Vorratsbeutel und dem Abflußbeutel verworfen. Das am Patienten verbleibende Kupplungsteil bzw. das Konnektionsstück wird in der Regel sicherheitshalber nochmals mit einer Schraubkappe verschlossen. Erst bei einer erneuten Anordnung wird der patientenseitige Schlauch abgeklemmt, das komplette Kupplungsteil entfernt und ein neuer Konnektor mit Kupplungsteil aufgeschraubt. Erst beim zweiten Schritt des Auslaufens wird die Klemme geöffnet.

Mit dieser Vorrichtung kann durch die ohnehin erfolgende Drehbewegung das Verschlußstück auf das Kupplungsteil aufgebracht werden und somit eine Kontamination weitgehend ausgeschlossen werden.

Eine Ausgestaltung der Erfindung besteht darin, daß das Gehäuse eine zylindrische Form aufweist und daß die drehbare Anordnung als Mittel zum Abdichten der Stutzen zusätzlich ein mit einem drehbaren Griffstück verbundenes, ebenfalls drehbares zylindrisches Dichtungsteil mit seitlichen Ausnehmungen aufweist. Hierdurch können jeweils zwei Stutzen in Fluidverbindung gebracht werden.

Durch die Mittel zum Abdichten kann die Durchflußrate regelbar sein.

Es ist auch erfindungsgemäß, daß jeweils zwei Stutzen zum Durchleiten eines Fluids gleichzeitig zu öffnen sind.

Es liegt im Rahmen der Erfindung, daß die Mittel zum Übertragen einer radialen Bewegung als Steuerkurve an der drehbaren Anordnung angeordnet sind.

Es kann vorgesehen sein, daß das Verschlußstück mit den Mitteln zum Übertragen einer radialen Bewegung formschlüssig verbindbar ist.

Eine erfindungsgemäße Weiterbildung sieht vor, daß die drehbare Anordnung auch Mittel zum Abziehen des Verschlußstucks von dem Kupplungsteil oder dem Konnektionsstück aufweist. Die Mittel zum Abziehen des Verschlußstücks von dem Kupplungsteil oder dem Konnektionsstück können eine mit der drehbaren Anordnung verbundene Steuerkurve aufweisen.

Das Mittel zum Abziehen bewirkt, daß patientenseitig nicht der Schlauch abgeklemmt und der Verschluß manuell entfernt werden muß, bevor ein neuer Konnektor angeschraubt wird.

Nach Konnektion bewirkt ein ebenfalls durch den Drehvorgang bewegtes Rückziehglied die Entfernung des Verschlußstücks kurz vor der Phase des Entleerens der Bauchhöhle in den Abfallbehälter.

Auch in dieser Ausführungsform muß bei jedem Verfahrensschritt eine eindeutige Zuordnung der Konnektorstellung erfolgen. So ist es mittels einer Arretiereinrichtung ausgeschlossen, daß nach Eindrücken des Verschlußstücks ein Weiterdrehen erfolgen und versehentlich der Stopfen wieder entnommen werden könnte.

Vorteilhafterweise ist der Konnektor zu Beginn der Peritonealdialysebehandlung an das Kupplungsteil mit einer Schutzhaube angeschlossen, sowie an einen gefüllten Vorratsbeutel mit frischer Dialyselösung und einen Leerbeutel zur Aufnahme verbrauchter Dialyselösung. Das gesamte System ist in sich geschlossen und sterilisiert. Da die Beutel in der Regel bereits angebracht sind, ist eine Verwechslungsgefahr ausgeschlossen und die Verfahrensschritte sind vorgegeben.

Es ist auch vorteilhaft, daß ein weiterer Verschluß über ein in dem Kupplungsteil befindliches Verschlußstück stülpbar und verschließbar ist.

Ebenso sieht eine Ausführungsform Mittel zum Vermeiden des versehentlichen Lösens des Konnektors vom Kupplungsteil und/oder des Kupplungsteils von einem Bauchanschluß des Patienten vor, welche beispielsweise als Überwurfmutter ausgebildet sein können.

Weiterhin ist vorgesehen, daß die drehbare Anordnung nach einmaligem Drehen arretierbar ist.

Da die Reihenfolge der Verfahrensschritte unabdingbar sein muß, ist erfindungsgemäß vorgesehen, daß der Konnektor nur in einer Drehrichtung drehbar ist und daß jeder Verfahrensschritt optisch (z.B. durch eine Fließrichtungsanzeige am Drehgriff) und/oder taktil, z.B durch eine innere Steuerkurve mit Einrastelementen, verfolgt werden kann. Desweiteren ist vorgesehen, daß immer nur zwei Kanäle miteinander verbindbar sind.

Erfindungsgemäß ist die Verwendung der Vorrichtung als Konnektor für die Peritonealdialyse. Im folgenden wird die Erfindung anhand von Zeichnungen, die eine beispielhafte Ausgestaltung der Erfindung wiedergeben, erläutert. Es zeigen
- Fig. 1: eine senkrecht geschnittene Darstellung einer erfindungsgemäßen Vorrichtung,
- Fig. 2: eine horizontal geschnittene Darstellung einer erfindungsgemäßen Vorrichtung,
- Fig. 3 bis Fig. 9: teilgeschnittene Darstellungen der Vorrichtung in verschiedenen Stadien eines Beutelwechsels in der Peritonealdialyse,
- Fig. 10: eine senkrecht geschnittene Darstellung einer erfindungsgemäßen Vorrichtung mit Mitteln zum Abziehen des Verschlußstücks von dem Kupplungsteil,
- Fig. 11: eine Ausführungsform der Erfindung, bei der die Stutzen mittels Verschlußstücken abgedichtet werden.

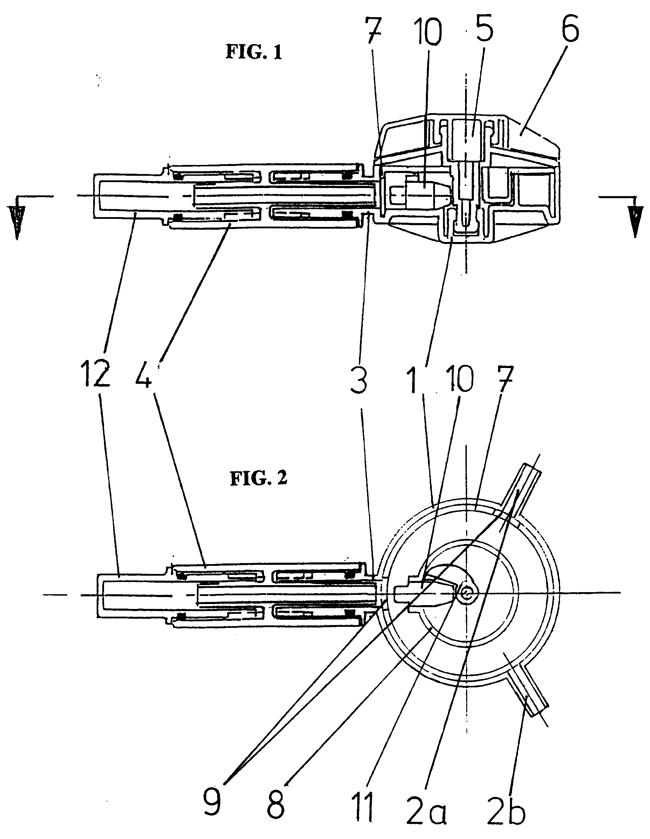

Wie in Fig. 1 und 2 dargestellt, weist die erfindungsgemäße Vorrichtung ein Gehäuse 1 mit zwei Stutzen 2a, 2b zur festen Aufnahme von jeweils einem Schlauchabschnitt auf. An dem Stutzen 2b würde bei der Anwendung in der Peritonealdialyse ein Beutel mit frischer Dialyselösung angeschlossen werden, an dem Stutzen 2a ein Leerbeutel zur Aufnahme der verbrauchten Dialyselösung.

Weiterhin weist die Vorrichtung einen weiteren Stutzen 3 auf, der zur Aufnahme eines Kupplungsteils 4 dient. Die Verbindung zwischen dem Stutzen 3 und dem Kupplungsteil 4 kann beispielsweise über eine mit O-Ringen abgedichtete Einrastverbindung erfolgen. Das Gehäuse, das vorzugsweise zylindrisch ausgebildet ist, weist eine drehbare Anordnung 5 zum Steuern des Flüssigkeitslaufes auf, die aus einem Griffstück 6 und einem mit dem Griffstück 6 verbundenen Dichtungsteil 7, das vorzugsweise ebenfalls zylindrisch ist, besteht und beispielsweise über ein axial verlaufendes hohlzylindrisches Element 8 mit dem Gehäuse 1 verbunden ist. Zweckmäßigerweise ist der äußere, vom Benutzer gehandhabte Teil, das Griffstück 6, dieser drehbaren Anordnung 5 so ausgebildet, daß er eine symbolische Darstellung des Flüssigkeitsverlaufes aufweist. Griffstück 6 und Dichtungsteil 7 greifen z.B. formschlüssig ineinander.

Das Dichtungsteil 7 weist zwei Ausnehmungen 9 auf, die je nach Stellung des Dichtungsteils 7 entweder eine flüssigkeitsdurchgängige Verbindung zwischen jeweils zwei Stutzen 2a, 2b, 3 des Gehäuses 1 herstellen oder flüssigkeitsdicht abdichten. Die Abdichtung zum Griffstück 6 hin erfolgt beispielsweise über die Deckplatte des Dichtungsteils 7, die auf dem hohlzylindrischen Element 8 federnd aufliegt.

Innerhalb des Gehäuses 1, beispielsweise in der Wand des hohlzylindrischen Elements 8, ist ein Verschlußstück 10 angeordnet, das geeignet ist, das Kupplungsteil 4 bzw. direkt das Konnektionsstück flüssigkeitsdicht zu verschließen. Dieses Verschlußstück 10 ist vorzugsweise in Richtung der Längsachse des Kupplungsteils 4 bzw. des Konnektionsstücks angeordnet.

Die drehbare Anordnung 5 weist Mittel 11 zum Übertragen einer radialen Vorschubbewegung auf das Verschlußstück 10 auf, die beispielsweise innerhalb des hohlzylindrischen Elements 8 an dem Dichtungsteil 7 angeordnet und mit dem drehbaren Griffstück verbunden sind. Es kann sich bei diesen Mitteln beispielsweise um eine Steuerkurve handeln.

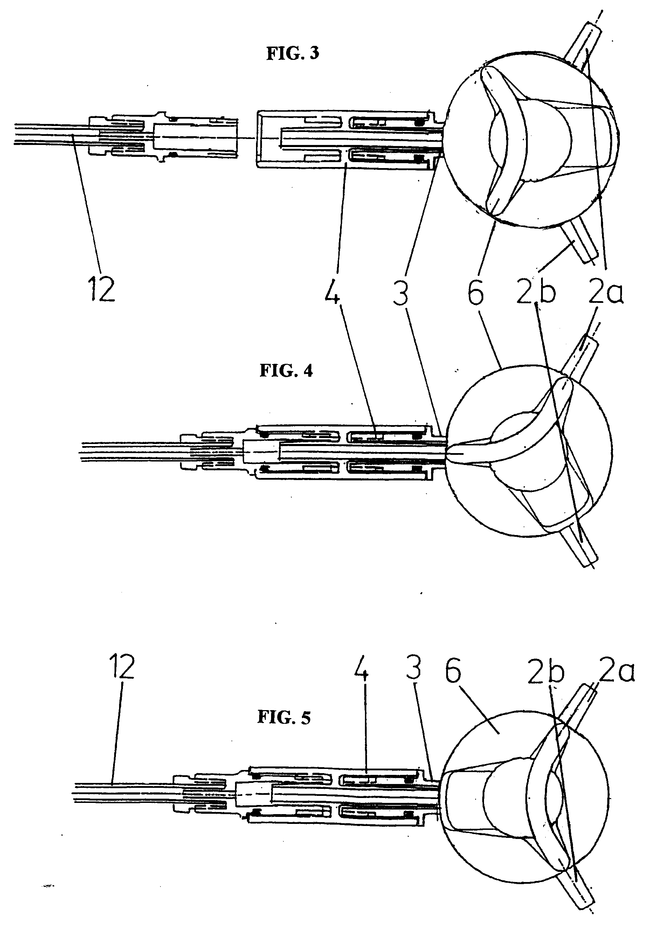

Die Fig. 3 bis 9 zeigen den Ablauf eines Beutelwechsels in der Peritonealdialyse mit der erfindungsgemäßen Vorrichtung.

Zunächst wird die mit einem Kupplungsteil 4 versehene Vorrichtung mit einem Bauchkatheter 12 verbunden (Fig. 3). Anschließend (Fig. 4) wird die verbrauchte Dialyseflüssigkeit aus dem Bauchraum durch die Vorrichtung und den Stutzen 2a in einen Beutel geleitet, in dem die verbrauchte Dialyseflüssigkeit gesammelt wird. Dann wird die drehbare Anordnung 5 um eine Position weitergedreht und die Vorrichtung mit frischer Dialyselösung, die von dem Stutzen 2b durch die Vorrichtung und den Stutzen 2a in den Beutel mit verbrauchter Dialyselösung läuft, gespült (Fig. 5). Dabei ist selbstverständlich der Patientenzugang verschlossen.

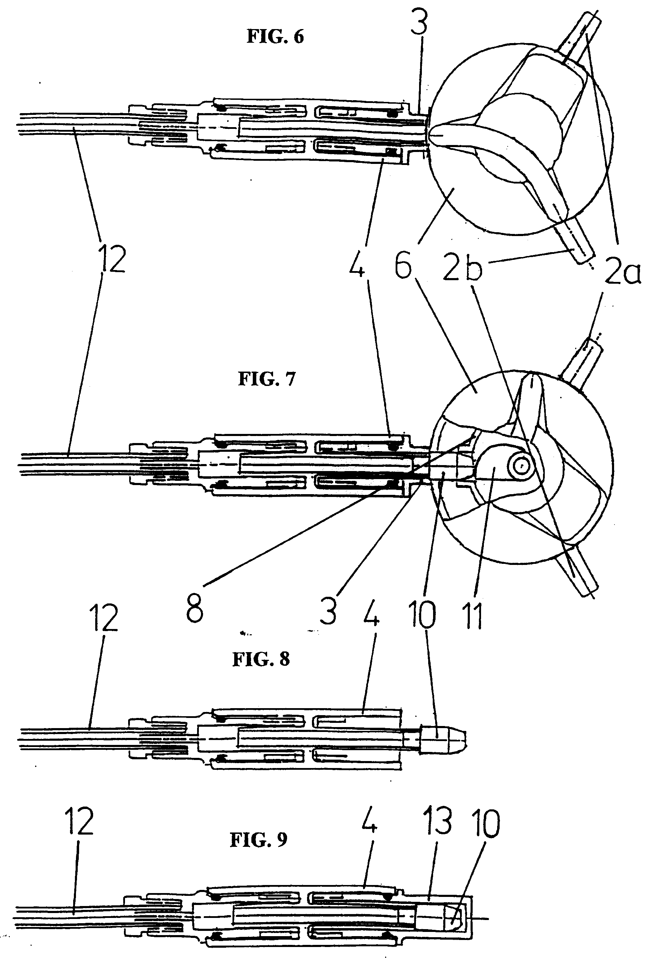

Nun erfolgt eine weitere Drehung der drehbaren Anordnung 5 und der Patient wird über den Bauchkatheter mit frischer Dialyselösung versorgt (Fig. 6).

Im folgenden Schritt wird durch Weiterdrehen der drehbaren Anordnung 5 jeglicher Flüssigkeitsdurchtritt durch das Gehäuse 1 unterbunden. Gleichzeitig ist das jeweilige Mittel 11 zum Übertragen einer radialen Vorschubbewegung an das in dem hohlzylindrischen Element 8 angeordnete Verschlußstück 10 gestoßen und drückt dieses über seinen abgeschrägten bzw. konischen Bereich beim Weiterdrehen in die entsprechend ausgebildete Öffnung des Kupplungsteils 4 bzw. des Konnektionsstücks, bis dieses verschlossen ist (Fig. 7).

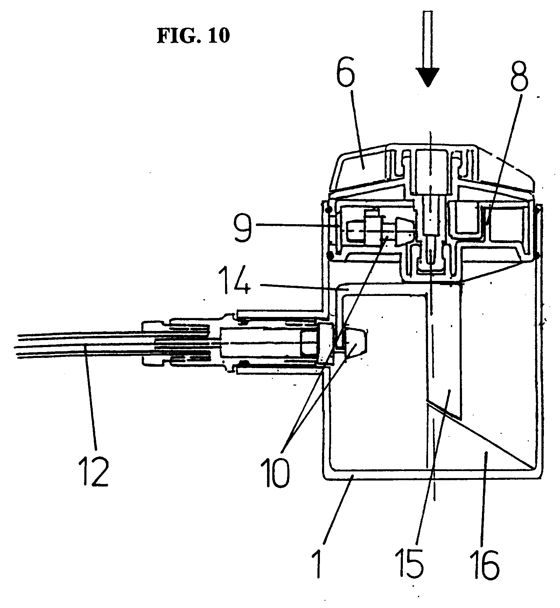

Nun kann das Gehäuse 1 von dem Kupplungsteil 4, das flüssigkeitsdicht verschlossen ist, abgenommen werden (Fig. 8). Schließlich kann noch ein Verschluß 13 über das Verschlußstück 10 gestülpt und festgeschraubt werden (Fig. 9).

Fig. 10 zeigt schließlich noch ein Beispiel für eine erfindungsgemäße Vorrichtung, die auch Mittel 14 zum Abziehen des Verschlußstücks 10 von dem Kupplungsteil 4 ausweist. Diese Mittel 14 bestehen aus einem mit der drehbaren Anordnung 5 verbundenen Rückziehglied 15, das hinter den Kopf des Verschlußstücks 10 greift, wodurch das Verschlußstück 10 wieder entfernt werden kann.

Durch Druck auf die Vorrichtung (in Pfeilrichtung) wird das Rückziehglied 15 auf der schrägen Ebene 16 laufend nach unten gedrückt und bewegt sich daher nach rechts, wobei es das Verschlußstück 10 mitnimmt. Durch weiteren Druck gelangt die hier im Gehäuse 1 axial verschiebbar angeordnete drehbare Anordnung 5 vor die Öffnung des zum Kupplungsteil 4 gehörenden Stutzen 3. Danach kann der Vorgang wie oben beschrieben erneut ablaufen.

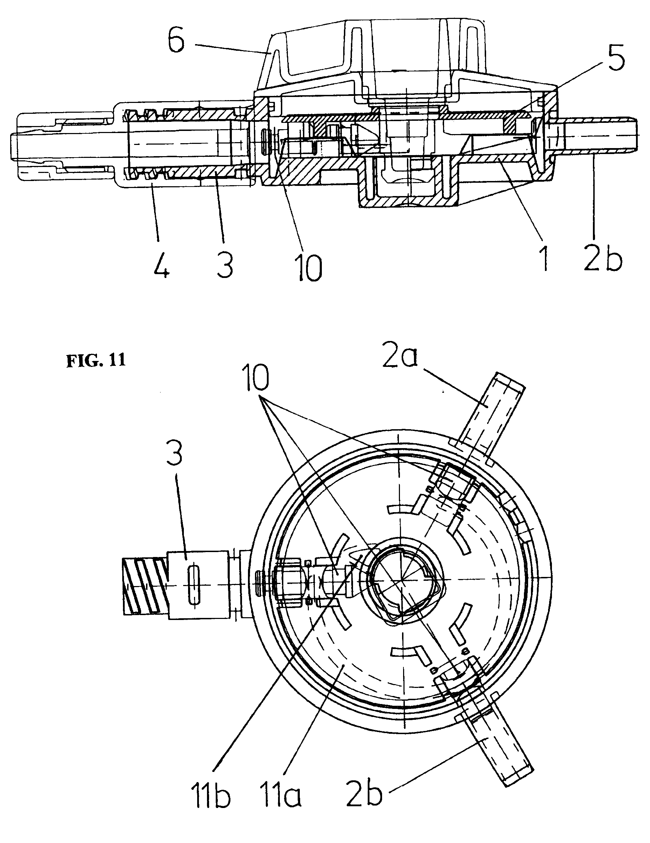

In Fig. 11 ist schließlich eine weitere Ausführung der Erfindung in einer geschnittenen Seitenansicht und einer geschnittenen Draufsicht dargestellt, bei der die Stutzen 2a, 2b, 3 mittels Verschlußstücken 10 verschlossen werden. Das drehbare Dichtungsteil 7 entfallt somit. Wie dargestellt, liegen im Bereich der Stutzen 2a, 2b, 3 Verschlußstücke vor, die in Bezug auf das Gehäuse in radialer Richtung in einer Führung verschiebbar sind. Als Mittel 11 zum Übertragen einer radialen Bewegung ist hier eine gestrichelt dargestellte kreisbogenförmige Steuerkurve 11a vorgesehen, die mit den Verschlußstücken 10 durch eine in diesen vorliegende Nut formschlüssig verbunden ist. Die Mittel 11 zum Übertragen einer radialen Bewegung sind über die drehbare Anordnung 5 mit dem drehbaren Griffstück 6 verbunden. Durch Drehen des drehbaren Griffstücks 6 werden somit die Verschlußstücke 10 in radialer Richtung verschoben, wobei sie die Stutzen 2a, 2b und 3 in der selben Reihenfolge öffnen und schließen, wie es durch das Drehen des Dichtungsteils 7 in den Fig. 3 bis 9 dargestellt ist. Somit ist in diesem Fall das Mittel 11 zum Übertragen einer radialen Bewegung auf das Verschlußstück 10, nämlich die Steuerkurve 11a, identisch mit dem Mittel 14 zum Abziehen des Verschlußstücks 10 von dem Kupplungsteil 4. Am Ende des Beutelwechsels wird das das Kupplungsteil 4 oder das Konnektionsstück verschließende Verschlußstück 10 entweder über die kreisbogenförmige Steuerkurve 11a oder über die keilförmige Steuerkurve 11b oder beide in das Kupplungsteil 4 oder das Konnektionsstück hineingedrückt, wodurch es dicht verschlossen wird.

## Patentansprüche

1. Vorrichtung zum Steuern eines Fluidverlaufes, bestehend aus einem Gehäuse (1) mit mindestens einem Stutzen (2a, 2b) zur festen Ankupplung eines Schlauchabschnitts und mindestens einem weiteren Stutzen (3) zur Aufnahme eines Kupplungsteiles (4), wobei die Verbindung zwischen den weiteren Stutzen (3) und dem Kupplungsteil (4) lösbar ist und wobei eine drehbare Anordnung (5) zum Steuern des Fluidverlaufes durch das Gehäuse (1) sowie Mittel zum Abdichten der Stutzen (2a, 2b, 3) vorgesehen sind, **dadurch gekennzeichnet**, daß die Mittel zum Abdichten der Stutzen (2a, 2b, 3) mindestens ein in dem Gehäuse (1) angeordnetes bewegliches Verschlußstück (10) beinhalten und daß die drehbare Anordnung (5) mindestens ein Mittel (11) zum Übertragen einer radialen Bewegung auf das Verschlußstück (10) aufweist.

2. Vorrichtung gemäß Anspruch 1, **dadurch gekennzeichnet**, daß das Gehäuse (1) eine zylindrische Form aufweist und daß die drehbare Anordnung (5) als Mittel zum Abdichten der Stutzen (2a, 2b, 3) zusätzlich ein mit einem drehbaren Griffstück (6) verbundenes, ebenfalls drehbares zylindrisches Dichtungsteil (7) mit seitlichen Ausnehmungen aufweist.

3. Vorrichtung gemäß Anspruch 1 oder 2, **dadurch gekennzeichnet**, daß durch die Mittel zum Abdichten die Durchflußrate der Flüssigkeit regelbar ist.

4. Vorrichtung gemäß Anspruch 3, **dadurch gekennzeichnet**, daß maximal zwei Stutzen (2a, 2b, 3) zum Durchleiten eines Fluids gleichzeitig zu öffnen sind.

5. Vorrichtung gemäß Anspruch 1, **dadurch gekennzeichnet**, daß als Mittel (11) zum Übertragen einer radialen Bewegung mindestens eine Steuerkurve an der drehbaren Anordnung (5) angeordnet ist.

6. Vorrichtung gemäß Anspruch 1, **dadurch gekennzeichnet**, daß das Verschlußstück (10) mit dem Mittel (11) zum Übertragen einer radialen Bewegung formschlüssig verbindbar ist.

7. Vorrichtung gemäß Anspruch 1, **dadurch gekennzeichnet**, daß die drehbare Anordnung auch Mittel (14) zum Abziehen des Verschlußstücks (10) von dem Kupplungsteil (4) aufweist.

8. Vorrichtung gemäß Anspruch 7, **dadurch gekennzeichnet**, daß die Mittel (14) zum Abziehen des Verschlußstücks (10) von dem Kupplungsteil (4) eine mit der drehbaren Anordnung (5) verbundene Steuerkurve umfassen.

9. Vorrichtung gemäß Anspruch 1, **dadurch gekennzeichnet**, daß ein weiterer Verschluß (13) über ein in dem Kupplungsteil (4) oder dem Konnektionsstück befindliches Verschlußstück (10) stülpbar und verschließbar ist.

10. Vorrichtung gemäß Anspruch 1, **dadurch gekennzeichnet**, daß Mittel zum Vermeiden des Lösens der Vorrichtung von dem Kupplungsteil (4) und/oder des Kupplungsteils (4) von einem Bauchanschluß vorgesehen sind.

11. Vorrichtung gemäß Anspruch 1, **dadurch gekennzeichnet**, daß die drehbare Anordnung (5) nach einmaligem Drehen arretierbar ist.

12. Vorrichtung gemäß Anspruch 1, **dadurch gekennzeichnet**, daß die drehbare Anordnung (5) nur in einer Drehrichtung drehbar ist und daß jeder Drehvorgang optisch und/oder taktil verfolgbar ist.

13. Verwendung der Vorrichtung gemäß einem der Ansprüche 1 bis 12 als Konnektor für die Peritonealdialyse.

## Claims

1. A flow sequence control device consisting of a housing (1) with at least one fitting (2a, 2b) to which a hose section can be firmly attached, and at least one additional fitting (3) for attachment of a coupling component (4), the connection between the additional fitting (3) and the coupling component (4) being releasable, and a rotatable arrangement (5) for controlling the flow sequence through the housing (1) being provided, as well as means to seal off the fittings (2a, 2b, 3), **characterized in that** the means for sealing off the fittings (2a, 2b, 3) contains at least one movable closure element (10) disposed in the housing (1), and that the rotatable arrangement (5) has at least one means (11) for transmitting radial movement to the closure element (10).

2. The device of claim 1, **characterized in that** the housing (1) is cylindrical and that the rotatable arrangement (5) which serves as means to seal off the fittings (2a, 2b, 3) is provided additionally with a likewise rotatable, cylindrical sealing element (7) with lateral recesses, which is connected with a rotatable handle (6).

3. The device of claim 1, **characterized in that** the flow rate of the fluid can be controlled with the sealing-off means.

4. The device of claim 3, **characterized in that** a maximum of two fittings (2a, 2b, 3) may be open at any one time for the passage of a liquid.

5. The device of claim 1, **characterized in that** at least one radial cam is disposed on the rotatable arrangement (5) as means (11) for transmitting radial movement.

6. The device of claim 1, **characterized in that** the closure element (10) can interlock with the means (11) for transmitting radial movement.

7. The device of claim 1, **characterized in that** the rotatable arrangement also has means (14) for pulling off the closure element (10) from the coupling component (4).

8. The device of claim 7, **characterized in that** the means (14) for pulling off the closure element (10) from the coupling component (4) includes a radial cam connected to the rotatable arrangement (5).

9. The device of claim 1, **characterized in that** an additional closing device (13) can be fitted over a closure element (10) in the coupling component (4) or in the connection piece, and can be locked in position.

10. The device of claim 1, **characterized in that** means are provided to prevent disconnection of the device from the coupling component (4) and/or of the coupling component (4) from an abdominal catheter.

11. The device of claim 1, **characterized in that** the rotatable arrangement (5) can be arrested after it has been advanced by one position.

12. The device of claim 1, **characterized in that** the rotatable arrangement (5) can only be rotated in one direction, and that each advance can be monitored optically and/or by tactile means.

13. Use of the device according to any of claims 1 to 12 as a connector in peritoneal dialysis.

## Revendications

1. Dispositif de contrôle du parcours d'un fluide, composé d'un boîtier (1) ayant au moins une tubulure (2a, 2b) destinée au raccordement fixe d'un segment de tuyau et au moins une tubulure supplémentaire (3) destinée à recevoir un raccord (4), la liaison entre la tubulure supplémentaire (3) et le raccord (4) pouvant être rompue, un dispositif tournant (5) destiné au contrôle du parcours du fluide à travers le boîtier (1) et des moyens d'obturation des tubulures (2a, 2b, 3) étant prévus, **caractérisé en ce que** les moyens d'obturation des tubulures (2a, 2b, 3) contiennent au moins un organe d'obturation (10) mobile positionné dans le boîtier (1) et en ce que le dispositif tournant (5) est muni d'au moins un moyen (11) destiné à transmettre un mouvement radial à l'organe d'obturation (10).

2. Dispositif selon la revendication 1, **caractérisé en ce que** le boîtier (1) présente une forme cylindrique et en ce que le dispositif tournant (5) est muni en outre d'un élément d'étanchéité (7) cylindrique tournant qui sert de moyen d'obturation des tubulures (2a, 2b, 3) et qui est relié à une poignée (6) également tournante, ledit élément d'étanchéité (7) présentant des évidements latéraux.

3. Dispositif selon la revendication 1 ou 2, **caractérisé en ce que** le débit du liquide peut être réglé à l'aide du moyen d'obturation.

4. Dispositif selon la revendication 3, **caractérisé en ce que** deux tubulures (2a, 2b, 3) au maximum peuvent être ouvertes simultanément pour laisser circuler un liquide.

5. Dispositif selon la revendication 1, **caractérisé en ce qu'au** moins une came de commande est placée dans le dispositif tournant (5) comme moyen (11) destiné à transmettre un mouvement radial.

6. Dispositif selon la revendication 1, **caractérisé en ce que** l'organe d'obturation (10) peut être relié au moyen (11) destiné à transmettre un mouvement radial en épousant sa forme.

7. Dispositif selon la revendication 1, **caractérisé en ce que** le dispositif tournant est muni également de moyens (14) pour retirer l'organe d'obturation (10) du raccord (4).

8. Dispositif selon la revendication 7, **caractérisé en ce que** les moyens (14) pour retirer l'organe d'obturation (10) du raccord (4) comprennent une came de commande reliée au dispositif tournant (5).

9. Dispositif selon la revendication 1, **caractérisé en ce qu**'un organe obturateur supplémentaire (13) peut être enfilé et verrouillé sur un organe d'obturation (10) situé dans le raccord (4) ou l'élément de connexion.

10. Dispositif selon la revendication 1, **caractérisé en ce qu**'il est prévu des moyens pour éviter que le dispositif ne se sépare du raccord (4) et / ou que le raccord (4) ne se sépare d'un raccordement ventral.

11. Dispositif selon la revendication 1, **caractérisé en ce que** le dispositif tournant (5) peut être arrêté après avoir effectuer un tour.

12. Dispositif selon la revendication 1, **caractérisé en ce que** le dispositif tournant (5) ne peut tourner que dans un sens et en ce que chaque mouvement de rotation peut être suivi optiquement et / ou tactilement.

13. Utilisation du dispositif selon l'une des revendications 1 à 12 comme connecteur pour la dialyse péritonéale.
